# EUROPEAN PATENT SPECIFICATION

(11) **EP 1 189 620 B1**
(45) Date of publication and mention of the grant of the patent: **23.06.2004**
(21) Application number: 00920588.1
(22) Date of filing: 27.03.2000
(51) Int. Cl.: A61K 31/568, A61K 47/44

(54) **FORMULATION COMPRISING TESTOSTERON UNDECANOATE AND CASTOR OIL**
FORMULIERUNG ENTHALTEND TESTOSTEONUNDECANOAT UND RIZINUSÖL
PREPARATION COMPRENANT DE LA TESTOSTERONE UNDECANOATE ET DE L'HUILE DE RICIN

(30) Priority: 01.04.1999 EP 99302581
(43) Date of publication of application: 27.03.2002
(73) Proprietor: Akzo Nobel N.V., 6824 BM Arnhem (NL)
(72) Inventor: NIJS DE, Henrik, NL-5343 XC OSS (NL); CHANDLER, Susan, Marlsborough, Wiltshire (GB); PERRY, Elizabeth, Anne, Swindon, Highworth SN6 7BJ (GB); FERDINANDO, Josephine Joan Christine, Monkton Park, Chippenham SN15 3XW (GB)
(74) Representative: Kraak, Hajo
(86) International application number: PCT/EP2000/002677
(87) International publication number: WO 2000/059512

(56) References cited:
- WO-A-95/12383
- WO-A-97/40823
- WO-A-98/36770
- CHEMICAL ABSTRACTS, vol. 107, no. 13, 28 September 1987 (1987-09-28) Columbus, Ohio, US; DAVIDSON D W ET AL: "Increasing circulating androgens with oral testosterone undecanoate in eugonadal men" page 91; XP002082921 & DAVIDSON D W ET AL: "Increasing circulating androgens with oral testosterone undecanoate in eugonadal men" J STEROID BIOCHEM, vol. 26, no. 6, 1987, pages 713-715, ENGLAND

## Description

The invention is in the field of androgen formulations for oral administration. Such formulations have acquired new attention in view of the development of preparations for male contraception and male HRT (hormone replacement therapy). In both cases the androgen may particularly be used as a replacement for endogenic testosterone. Thus, e.g., in male HRT, androgen is administered in order to relieve the undesired effects of the (partial) androgen-deficiency which may result of age. Androgens can also be used in the female, e.g. as androgen replacement therapy in postmenopausal women.

Oral preparations of androgens are rare, the only oral natural testosterone product available on the market being a solution of testosterone undecanoate (TU) in oleic acid. This product, which is known in various countries under various tradenames, e.g. Andriol® or Restandol®, is a soft-gelatine capsule formulation containing 40 mg of TU dissolved in oleic acid. To achieve and maintain acceptable testosterone levels in the blood, 3-4 such capsules should be administered daily. A regimen involving such a large number of separate administrations is not very suitable for the practical use of TU as an acceptable HRT product, let alone that it would be accepted this way in contraception.

The invention seeks to solve the problem of providing an orally active androgen formulation which is well absorbed in the human body. Particularly, the invention seeks to provide a formulation of TU which has a higher strength than the known TU formulation referred to above.

To meet these and other objectives, the invention provides a pharmaceutical formulation in the form of a capsule for oral administration comprising testosterone undecanoate as an active ingredient dissolved in a pharmaceutically acceptable liquid carrier, wherein the liquid carrier is castor oil. Although, in one embodiment, castor oil can be the sole liquid carrier, it is also possible to combine castor oil with other liquid carriers. It is desired, however, that castor oil is the main component of the liquid carrier, i.e. it makes up more than 50% by weight of said carrier.

The choice of castor oil as the liquid carrier, in conjunction with the choice of testosterone undecanoate as the androgen, makes for a solution which can comprise 200-250 mg of TU per ml. This amounts to 127 to 137 mg of testosterone per ml. This is a novel achievement for any orally administerable solution of testosterone in any form. The invention, in one aspect, therefore includes a pharmaceutical formulation in the form of a capsule for oral administration comprising testosterone undecanoate as the active ingredient dissolved in a pharmaceutically acceptable liquid carrier in a concentration of 200-250 mg/ml.

It is noted that Exp.Clin.Endocrinol.Diabetis 105 Suppl.1,21,1997 and Eur. J. Endocrino1.123:514-9 (1995) make mention of an injectable solution of 250 mg/ml of TU in castor oil. This, however, does not at all lead the way to the possibility of achieving oral administration of a similar level of TU. These are separate fields of pharmaceutical formulation, and, moreover, an injection directly into the muscle cannot be used as a model for administration via the oral route. In addition, it is all the more surprising that notably castor oil is a suitable carrier for this type of administration, since its normal capacity is that of a laxative. This, of course, is a use completely opposite to that underlying the present invention, wherein the goal is to make something (in this case TU) enter and be absorbed into the human body rather than make it leave the body by inducing excretion. Hence it is fully unexpected that castor oil turns out to be a highly suitable carrier for the oral administration of TU, which is absorbed well.

The invention also includes the use of testosterone undecanoate for the manufacture of a medicine in the form of an oral solution, wherein testosterone undecanoate is dissolved in a pharmaceutically acceptable liquid carrier, characterised in that the liquid carrier comprises at least 50% by weight of castor oil. In order to achieve optimal administration of TU, the capsules should be taken preferably during or just after a meal.

In all of the above aspects, it is preferred that, in addition to the TU and the castor oil, the capsules include additives such as disclosed in WO 97/40823 and WO 95/24893. It is most preferred to base the formulation on a particular combination of castor oil and a lipophilic surfactant, as such a combination provides a liquid vehicle for dissolving TU resulting in the most stable formulations which best promote lymphatic absorption. Thus the preferred formulation is one in which TU is dissolved in a liquid vehicle which comprises of from 50 to 70% by weight of castor oil, and from 30 to 50% by weight of a lipophilic surfactant having an HLB <10, and, optionally, from 0 to 20% by weight of a hydrophilic surfactant, wherein the liquid vehicle is substantially free from the free fatty acid and contains less than 10% by weight of ethanol. Suitable lipophilic surfactants having an HLB <10 are known to the person skilled in the art. These include mono- and/or diglycerides of fatty acids as well as mono- and/or diglycerides of fatty acids in which the remaining free OH groups of glycerol can be esterified, such as the acetic, succinic, lactic, citric and/or tartaric esters thereof; propylene glycol mono- and/or di-esters of fatty acids; ethoxylates of castor oil or of hydrogenated castor oil (low ethoxylate content, HLB < 10); acid and ester ethoxylates formed by reacting ethylene oxide with fatty acids or glycerol esters of fatty acids; sorbitan esters of fatty acids; unsaturated polyglycolised glycerides (if HLB < 10); alcohol ethoxylates (if HLB <10); polyoxyethylene-polyoxypropylene co-polymers and block copolymers (if HLB < 10). The lipophilic surfactant is preferably used in an amount in the range of from 35 to 45% by weight of the liquid vehicle. A preferred lipophilic surfactant is lauroglycol (propylene glycol monolaurate). The optional hydrophilic surfactants are also known to the person skilled in the art. Any pharmaceutically acceptable hydrophilic surfactant (i.e., having an HLB value greater than 10) may be used in the present invention. The formulations may contain minor amounts of other additives, e.g., anti-oxidants, such as d-α-tocopherol, BHA, BHT; co-solvents such as ethanol and Transcutol (diethylene glycol monoethylether), plasticisers, such as propylene glycol, etc.

The formulations of the invention may readily be prepared by known methods, e.g. as described in WO 95/24893. The capsules encapsulating the formulations can be made by known techniques. Gelatine softgels, as with Andriol®, are preferred, but the wall of the capsule may be made from any pharmaceutically acceptable softshell or hardshell material. Methods of softgel encapsulation are disclosed in Theory and Practice of Industrial Pharmacy - Lachman & Leibermann, 2^{nd} Edition, published by Henry Kimpton Publishers, London. Methods of liquid-fill hardshell encapsulation are disclosed in Hardcapsules - Development and Technology - Edited by K. Ridgeway, published by Pharmaceutical Press 1987.

The invention will be further illustrated hereinafter with reference to the Example.

### EXAMPLE

The following ingredients are added to a suitable mixer (Unimix) and heated to 40°C:

| | |
|---|---|
| Castor oil BP | 53 parts by weight |
| Lauroglycol FCC | 35 parts by weight |
| Testosteron undecanoate | 12 parts by weight |

After complete dissolution the resulting solution is filled into soft gelatine capsules of 330 ml using standard equipment. A stable formulation is obtained, from which TU is absorbed well orally.

## Claims

1. A pharmaceutical formulation in the form of a capsule for oral administration comprising testosterone undecanoate as an active ingredient dissolved in a pharmaceutically acceptable liquid carrier, **characterised in that** the liquid carrier comprises at least 50% by weight of castor oil.

2. A pharmaceutical formulation according to claim 1, **characterised in that** the sole liquid carrier is castor oil.

3. A pharmaceutical formulation according to claim 1, **characterised in that** the liquid carrier further comprises from 30 to50% by weight of a lipophilic surfactant having an HLB value below 10.

4. A pharmaceutical formulation according to claim 3, **characterised in that** the lipophilic surfactant is lauroglycol.

5. A pharmaceutical formulation according to any one of the preceding claims, **characterised in that** testosterone undecanoate is dissolved in a concentration of 200-250 mg/ml.

6. The use of testosterone undecanoate for the manufacture of a medicine in the form of an oral solution, wherein testosterone undecanoate is dissolved in a pharmaceutically acceptable liquid carrier, **characterised in that** the liquid carrier comprises at least 50% by weight of castor oil.

7. Use of a pharmaceutical formulation according to claim 1 for the manufacture of a medicament for treatment of a human male or female in need of androgen supplementation.

## Patentansprüche

1. Pharmazeutische Formulierung in Form einer Kapsel zur oralen Verabreichung umfassend Testosteronundecanoat als ein Wirkstoff, gelöst in einem pharmazeutisch verträglichen, flüssigen Träger, **dadurch gekennzeichnet, dass** der flüssige Träger mindestens 50 Gew.-% an Rizinusöl umfasst.

2. Pharmazeutische Formulierung gemäss Anspruch 1, **dadurch gekennzeichnet, dass** Rizinusöl der einzige flüssige Träger ist.

3. Pharmazeutische Formulierung gemäss Anspruch 1, **dadurch gekennzeichnet, dass** der flüssige Träger ferner von 30 bis 50 Gew.-% eines lipophilen Tensides mit einem HLB-Wert von unter 10 umfasst.

4. Pharmazeutische Formulierung gemäss Anspruch 3, **dadurch gekennzeichnet, dass** das lipophile Tensid Lauroglycol ist.

5. Pharmazeutische Formulierung gemäss einem der vorangehenden Ansprüche, **dadurch gekennzeichnet, dass** das Testosteronundecanoat in einer Konzentration von 200-250 mg/ml gelöst ist.

6. Verwendung von Testosteronundecanoat zur Herstellung eines Arzneimittels in Form einer oralen Lösung, worin das Testosteronundecanoat in einem pharmazeutisch verträglichen flüssigen Träger gelöst ist, **dadurch gekennzeichnet, dass** der flüssige Träger mindestens 50 Gew.-% an Rizinusöl umfasst.

7. Verwendung einer pharmazeutischen Formulierung gemäss Anspruch 1 in der Herstellung eines Medikamentes zur Behandlung eines einer Androgen-Supplementierung bedürftigen, männlichen oder weiblichen Mensches.

## Revendications

1. Une formulation pharmaceutique sous forme d'une gélule pour administration orale comprenant de l'undecanoate de testosterone comme principe actif dissout dans un diluant support liquide pharmaceutiquement acceptable, **caractérisée en ce que** le diluant comprend au moins 50% en poids d'huile de ricin.

2. Une formulation pharmaceutique selon la revendication 1, **caractérisée en ce que** l'unique diluant liquide est l'huile de ricin.

3. Une formulation pharmaceutique selon la revendication 1, **caractérisée en ce que** le diluant comprend en plus, de 30 à 50% en poids d'un tensio-actif lipophile ayant une HLB inférieure à 10.

4. Une formulation pharmaceutique selon la revendication 3, **caractérisée en ce que** le tensio-actif est le lauroglycol.

5. Une formulation pharmaceutique selon l'une quelconque des revendications précédentes, **caractérisée en ce que** l'undécanoate de testostérone est dissous à raison d'une concentration de 200-250 mg/ml.

6. Utilisation de l'undécanoate de testostérone pour la préparation d'un médicament sous forme de solution orale, dans laquelle l'undécanoate de testostérone est dissous dans un diluant liquide pharmaceutiquement acceptable, **caractérisé en ce que** le diluant comprend au moins 50% en poids d'huile de ricin.

7. Utilisation d'une formulation pharmaceutique selon la revendication 1 pour la préparation d'un médicament destiné au traitement d'un être humain de sexe masculin ou féminin nécessitant un apport d'androgène.
